# EUROPÄISCHE PATENTSCHRIFT

(11) **EP 4 623 985 B1**
(45) Veröffentlichungstag und Bekanntmachung des Hinweises auf die Patenterteilung: **04.11.2026**
(21) Anmeldenummer: 25165049.5
(22) Anmeldetag: 20.03.2025
(51) Int. Cl.: A61N 1/05, A61N 1/36

(54) **NEUROSTIMULATIONSKATHETER**
NEUROSTIMULATION CATHETER
CATHÉTER DE NEUROSTIMULATION

(30) Priorität: 27.03.2024 DE 102024108690
(43) Veröffentlichungstag der Anmeldung: 01.10.2025
(73) Patentinhaber: B. Braun Melsungen AG, 34212 Melsungen (DE)
(72) Erfinder: Wildhagen, Jens, 30659 Hannover (DE)
(74) Vertreter: Patentanwälte Ruff, Wilhelm, Beier, Dauster & Partner mbB

(56) Entgegenhaltungen:
- US-A- 6 059 779
- US-A1- 2006 020 317
- US-A1- 2016 279 406

## Beschreibung

Die Erfindung betrifft einen Neurostimulationskatheter mit den oberbegrifflichen Merkmalen des Anspruchs 1.

Die US 2006/020317 A1 offenbart unterschiedliche Katheter mit den oberbegrifflichen Merkmalen des Anspruchs 1. Bei einem der dort beschriebenen Katheter ist das Verankerungselement aus einem biologisch abbaubaren Material gefertigt. Bei einem weiteren der dort beschriebenen Katheter ist das Verankerungselement radial expandierbar und mittels eines Strukturelements abgedeckt. Das Strukturelement ist galvanisch auflösbar, um das darunterliegende Verankerungselement freizugeben und dessen Expansion zu ermöglichen.

Ein weiter Neurostimulationskatheter ist aus dem Stand der Technik bekannt und zur Verwendung bei einer Schmerztherapie mittels elektrischer Neurostimulation vorgesehen. Hierbei wird der längserstreckte Katheterschaft in das Körpergewebe eines Patienten eingeführt, wobei die an dem Katheterschaft angeordnete Elektrode im Bereich eines zu stimulierenden Nervs positioniert wird. Zur Hemmung der Erregungsweiterleitung des Nervs wird mittels der Elektrode ein elektrisches Stimulationssignal abgegeben. Durch die gehemmte Erregungsweiterleitung wird das Schmerzempfinden des Patienten gelindert oder unterdrückt. Dabei hat der Abstand zwischen Elektrode und Nerv maßgeblichen Einfluss auf die Effektivität der Schmerzunterdrückung.

Aufgabe der Erfindung ist es, einen Neurostimulationskatheter der eingangs genannten Art bereitzustellen, der eine verbesserte Schmerztherapie ermöglicht.

Diese Aufgabe wird durch das Bereitstellen eines Neurostimulationskatheters mit den Merkmalen des Anspruch 1 gelöst. Erfindungsgemäß ist vorgesehen, dass das Verankerungselement wenigstens abschnittsweise aus einem galvanischen Material gefertigt ist, wodurch das Verankerungselement unter Einwirkung eines elektrischen Gleichspannungssignals wenigstens abschnittsweise galvanisch auflösbar ist und damit die Verankerung an dem Körpergewebe lösbar ist. Durch die erfindungsgemäße Lösung wird eine einfache lösbare Verankerung des Katheterschafts am Körpergewebe des Patienten ermöglicht. Die Verankerung wirkt ungewollten Dislokationen des Katheterschafts und damit ungewollten Änderungen des Abstands zwischen der Elektrode und dem zu betäubenden Nerv entgegen. Hierdurch wird vermieden, dass die Effektivität der Schmerztherapie beeinträchtigt wird. Zu diesem Zweck ist das wenigstens eine Verankerungselement vorhanden. Um ein möglichst einfaches Lösen der Verankerung zu ermöglichen, ist das Verankerungselement wenigstens abschnittsweise aus dem galvanischen Material gefertigt. Das galvanische Material ist unter Einwirkung des elektrischen Gleichspannungssignals (galvanisch) auflösbar. Durch die wenigstens abschnittsweise Auflösung des Verankerungselements ist die Verankerung an dem Körpergewebe lösbar, beispielsweise indem das gesamte Verankerungselement galvanisch aufgelöst wird oder indem lediglich ein Abschnitt des Verankerungselements galvanisch aufgelöst wird. Durch die galvanisch lösbare Verankerung kann auf alternativ denkbare Mechanismen, Aktuatoren oder sonstige Einrichtungen zum Lösen des Verankerungselements verzichtet werden. Es versteht sich, dass auch mehrere erfindungsgemäß beschaffene Verankerungselemente vorhanden sein können. Das Verankerungselement selbst kann grundsätzlich jede für den vorliegenden Zweck geeignete Gestaltung aufweisen. Bei einer Ausgestaltung ist das Verankerungselement zur formschlüssigen Verankerung an dem Körpergewebe eingerichtet. Bei einer weiteren Ausgestaltung ist das Verankerungselement alternativ oder zusätzlich zur kraftschlüssigen Verankerung an dem Körpergewebe eingerichtet. Bei einer Ausgestaltung ist die wenigstens eine Stimulationselektrode an dem Katheterschaft ausgebildet. Mit anderen Worten: Der Katheterschaft selbst bildet gleichsam die wenigstens eine Stimulationselektrode. Bei einer weiteren Ausgestaltung ist die Stimulationselektrode längserstreckt und bildet gleichsam den Katheterschaft.

In Ausgestaltung der Erfindung ist das gesamte Verankerungselement aus dem galvanischen Material gefertigt. Unter Einwirkung des elektrischen Gleichspannungssignals löst sich folglich das gesamte Verankerungselement galvanisch auf. Im Vergleich zu einer lediglich abschnittsweisen galvanischen Auflösung des Verankerungselements wird vermieden, dass nicht aufgelöste Bestandteile des Verankerungselements im Körper des Patienten verbleiben. Zudem wird eine vergleichsweise einfache Fertigung erreicht, das das gesamte Verankerungselement aus ein- und demselben Material gefertigt werden kann.

In weiterer Ausgestaltung der Erfindung weist das Verankerungselement einen Verankerungsabschnitt und einen Befestigungsabschnitt auf. Der Verankerungsabschnitt ist zum Zusammenwirken mit dem Körpergewebe eingerichtet. Mit anderen Worten: Der Verankerungsabschnitt dient der eigentlichen form- und/oder kraftschlüssigen Verankerung an dem Körpergewebe. Der Befestigungsabschnitt dient der Befestigung des Verankerungselements an dem Katheterschaft. Dabei ist der Befestigungsabschnitt aus dem galvanischen Material gefertigt und der Verankerungsabschnitt ist aus einem resorbierbaren Material gefertigt. Unter Einwirkung des elektrischen Gleichspannungssignals wird folglich lediglich der Befestigungsabschnitt galvanisch aufgelöst. Hierdurch wird das Verankerungselement im Übrigen, im Speziellen der Verankerungsabschnitt, von dem Katheterschaft abgetrennt. Der Verankerungsabschnitt selbst wird hierbei nicht galvanisch aufgelöst und verbleibt zunächst im Körper des Patienten. Da der Verankerungsabschnitt aus dem erwähnten resorbierbaren Material gefertigt ist, wird er nach einer gewissen Zeit vom Körper des Patienten aufgenommen/aufgelöst (resorbiert). Dabei kommen grundsätzlich sämtliche für den vorliegenden Zweck geeignete resorbierbare Materialien infrage. Solche resorbierbaren Materialien sind der zuständigen Fachperson bekannt und müssen daher an dieser Stelle nicht näher spezifiziert werden. Indem lediglich der Befestigungsabschnitt aus dem galvanischen Material gefertigt wird, kann eine übermäßige Anreicherung von aufgelöstem Materials im Patientenkörper vermieden werden. Dies insbesondere im Vergleich zu einer gesamthaften Fertigung des Verankerungselements aus dem galvanischen Material.

In weiterer Ausgestaltung der Erfindung weist das galvanische Material Eisen, Nickel, Zinn, Zink und/oder Kupfer auf. Alternativ handelt es sich bei dem galvanischen Material um Eisen, Nickel, Zinn, Zink und/oder Kupfer. Die vorgenannten Materialien kommen als Spurenelemente im menschlichen Körper vor. Durch eine Auflösung der genannten galvanischen Materialien im Körper des Patienten sind daher keine gesundheitlichen Beeinträchtigungen zu befürchten, sofern die für das Verankerungselement verwendete Menge an Material und folglich eine sich nach dem galvanischen Auflösen ergebende Konzentration im Patientenkörper gewisse Grenzwerte einhält. Die Verwendung von Zink hat sich als besonders vorteilhaft erwiesen.

In weiterer Ausgestaltung der Erfindung ist das Verankerungselement eine längserstreckte Schraubwendel, die zum Einschrauben in das Körpergewebe eingerichtet ist. Zum Verankern des Katheterschafts wird die Schraubwendel um ihre Längsachse rotiert und hierdurch in das Körpergewebe eingeschraubt. Vorzugsweise ist die Schraubwendel an einem distalen Stirnende des Katheterschafts angeordnet und ragt koaxial in distaler Richtung von dem Katheterschaft ab. Zum Einschrauben der Schraubwendel kann folglich der gesamte Katheterschaft um seine Längsachse gedreht werden. Bei einer Ausgestaltung ist die gesamte Schraubwendel aus dem galvanisch auflösbaren Material gefertigt. Bei einer weiteren Ausgestaltung ist lediglich ein proximales Ende der Schraubwendel, mittels dessen die Schraubwendel an dem Katheterschaft befestigt ist, aus dem galvanisch auflösbaren Material gefertigt.

In weiterer Ausgestaltung der Erfindung ist das Verankerungselement ein Widerhakenelement, das zum Verhaken an dem Körpergewebe eingerichtet ist. Das Widerhakenelement wirkt einer proximal gerichteten Bewegung des Katheterschafts entgegen. Hierdurch wirkt das Widerhakenelement einem ungewollten Herausziehen des Katheterschafts aus dem Körpergewebe entgegen. Dies schließt nicht aus, dass das Widerhakenelement auch einem proximalen Vorschub des Katheterschafts entgegenwirkt. Bei einer Ausgestaltung ist das gesamte Widerhakenelement aus dem galvanisch auflösbaren Material gefertigt. Bei einer weiteren Ausgestaltung ist lediglich ein Befestigungsabschnitt des Widerhakenelements aus dem galvanischen Material gefertigt, wobei der Befestigungsabschnitt unmittelbar an dem Katheterschaft befestigt ist.

In weiterer Ausgestaltung der Erfindung ist das Widerhakenelement relativ zu dem Katheterschaft zwischen einer Einführposition und einer Verankerungsposition verlagerbar, wobei das Widerhakenelement in der Einführposition an dem Katheterschaft angelegt und in der Verankerungsposition von dem Katheterschaft abgespreizt ist. Eine solche Verlagerbarkeit des Verankerungselements zwischen der Einführ- und der Verankerungsposition erlaubt ein vereinfachtes Einführen des Katheterschafts in das Körpergewebe. Zu diesem Zweck ist das Widerhakenelement in der Einführposition an dem Katheterschaft angelegt. Mit anderen Worten: Das Widerhakenelement liegt eng an dem Katheterschaft an. In der Verankerungsposition ist das Widerhakenelement von dem Katheterschaft abgespreizt. Durch das Abspreizen des Widerhakenelements wird dessen eigentliche Widerhakenfunktion in Gang gesetzt. Mit anderen Worten: In der Verankerungsposition steht das Widerhakenelement nach außen von dem Katheterschaft ab und kann sich hierdurch kraft- und/oder formschlüssig an dem Körpergewebe verhaken. Bei einer Ausgestaltung ist das Verlagerungselement relativ zu dem Katheterschaft rotatorisch zwischen der Einführ- und der Verankerungsposition beweglich. Bei einer weiteren Ausgestaltung ist eine Schwenkbeweglichkeit gegeben. Zur entsprechend beweglichen Lagerung des Verankerungselements ist bei einer Ausgestaltung ein Drehlager vorgesehen. Bei einer weiteren Ausgestaltung ist ein Schwenklager vorgesehen, das im Speziellen als Festkörpergelenk gestaltet sein kann.

In weiterer Ausgestaltung der Erfindung ist das Widerhakenelement mittels eines Vorspannelements ausgehend von der Einführposition in Richtung der Verankerungsposition vorgespannt und mittels eines biokompatiblen Klebstoffs in der Einführposition fixiert, wobei der biokompatible Klebstoff unter Einwirkung einer physikalischen und/oder chemischen Zustandsgröße des Körpergewebes löslich ist. Diese Ausgestaltung der Erfindung erlaubt ein vereinfachtes Einführen des Katheters und dessen selbsttätige Verankerung nach dem Einführen. Zu diesem Zweck ist das wenigstens eine Widerhakenelement mittels des biokompatiblen Klebstoffs in der Einführposition fixiert. Hierdurch wird vermieden, dass das Widerhakenelement sich bereits während der Katheteranlage ungewollt in die Verankerungsposition verlagert. Nach der Katheteranlage/dem Einführen des Katheterschafts ist der biokompatible Klebstoff einer Einwirkung des Körpergewebes ausgesetzt. Um ein selbsttätiges Lösen der Fixierung zu ermöglichen, ist der biokompatible Klebstoff derart eingerichtet, dass er unter Einwirkung der erwähnten physikalischen und/oder chemischen Zustandsgröße des Körpergewebes löslich ist. Nach dem Lösen des biokompatiblen Klebstoffs bewirkt die Vorspannung des Widerhakenelements in Richtung der Verankerungsposition ein selbsttätiges Ausbilden der Verankerung. Dabei kann das Vorspannelement prinzipiell jede für den vorliegenden Zweck geeignete Gestaltung aufweisen. Beispielsweise kann das Vorspannelement durch einen elastischen Abschnitt des Katheterschafts und/oder des Verankerungselements gebildet sein. Alternativ oder zusätzlich kann das Vorspannelement als zusätzliches Bauteil ausgeführt sein, im Speziellen als Federelement, wobei unterschiedliche Bauformen denkbar sind (Spiralfeder, Drehfeder, Schenkelfeder oder dergleichen).

In weiterer Ausgestaltung der Erfindung ist die Zustandsgröße eine Feuchtigkeit, Fett, eine Temperatur und/oder ein pH-Wert. Folglich ist der biokompatible Klebstoff unter Einwirkung der Feuchtigkeit des Körpergewebes, des Körperfett, der Körpertemperatur und/oder eines pH-Werts des Körpergewebes löslich. Entsprechend eingerichtete biokompatible Werkstoffe sind einer auf dem Gebiet der medizinischen Klebetechnik tätigen Fachperson prinzipiell bekannt und müssen daher an dieser Stelle nicht in allen Einzelheiten spezifiziert werden.

In weiterer Ausgestaltung der Erfindung weist der biokompatible Klebstoff Zucker und/oder Stärke auf. Bei einer bevorzugten Ausgestaltung der Erfindung ist der biokompatible Klebstoff Zucker, im Speziellen aus Zuckermolekülen gebildet. Hierdurch ist der biokompatible Klebstoff auf besonders einfache und zuverlässige Weise unter Einwirkung der Feuchtigkeit des Körpergewebes auflösbar. Zudem ist durch den als Zucker aufgelösten Klebstoff keinerlei gesundheitliche Beeinträchtigung zu befürchten.

Die Erfindung betrifft zudem ein medizinisches System mit einem Neurostimulationskatheter gemäß der vorhergehenden Beschreibung und mit einem Signalgenerator. Der Signalgenerator ist mit dem Verankerungselement verbunden und zum Erzeugen des elektrischen Gleichspannungssignals eingerichtet. Die Verbindung zwischen dem Signalgenerator und dem wenigstens einen Verankerungselement dient der Übertragung des elektrischen Gleichspannungssignals zum wenigstens abschnittsweise galvanischen Auflösen des Verankerungselements. Diese Verbindung ist bei einer Ausgestaltung eine drahtgebundene Verbindung. Bei einer weiteren Ausgestaltung ist eine drahtlose Verbindung vorgesehen.

In weiterer Ausgestaltung der Erfindung ist der Signalgenerator mit der wenigstens einen Stimulationselektrode verbunden und zum Erzeugen eines gleichspannungsfreien Stimulationssignals eingerichtet. Das Stimulationssignal ist zum Hemmen der Erregungsweiterleitung des zu betäubenden Nervs eingerichtet. Bei dieser Ausgestaltung hat der Signalgenerator folglich eine Doppelfunktion und dient zum einen der Erzeugung des elektrischen Gleichspannungssignals und zum anderen zum Erzeugen des elektrischen Stimulationssignals. Da das Stimulationssignal gleichspannungsfrei ist, d. h. keine Gleichspannungsanteile aufweist, wird eine ungewollte Überlagerung mit dem Gleichspannungssignal vermieden. Im Speziellen wird vermieden, dass das galvanische Material des Verankerungselements sich unter Einwirkung des Stimulationssignals ungewollt auflöst und hierdurch die Verankerung vorzeitig aufgehoben wird.

Die Offenbarung betrifft zudem ein Verfahren zum Verankern und Lösen eines Neurostimulationskatheters. Das offenbarungsgemäße Verfahren weist die Schritte auf: Verankern eines Katheterschafts des Neurostimulationskatheters, wobei der Katheterschaft mittels eines Verankerungselements, das an dem Katheterschaft befestigt ist, an einem Körpergewebe verankert wird, das den Katheterschaft umgibt; Lösen der Verankerung, wobei ein elektrisches Gleichspannungssignal an das Verankerungselement angelegt wird, wobei wenigstens ein aus einem galvanischen Material gefertigter Abschnitt des Verankerungselements unter Einwirkung des Gleichspannungssignals galvanisch aufgelöst und damit die Verankerung an dem Körpergewebe gelöst wird. Die mit dem offenbarungsgemäßen Verfahren einhergehenden Vorteile korrespondieren mit den Vorteilen des erfindungsgemäßen Neurostimulationskatheters. Weitere Ausgestaltungen des offenbarungsgemäßen Verfahrens ergeben sich aus den Merkmalen der Ausgestaltungen des erfindungsgemäßen Neurostimulationskatheters und des medizinischen Systems. Zur Vermeidung von Wiederholungen wird daher auf die diesbezügliche Offenbarung verwiesen und ausdrücklich Bezug genommen.

Weitere Vorteile und Merkmale der Erfindung ergeben sich aus den Ansprüchen sowie aus der nachfolgenden Beschreibung bevorzugter Ausführungsbeispiele der Erfindung, die anhand der Zeichnungen dargestellt sind.
- Fig. 1: zeigt in schematisch vereinfachter Darstellung eine Ausführungsform eines erfindungsgemäßen medizinischen Systems mit einem Neurostimulationskatheter und einem Signalgenerator, wobei der Neurostimulationskatheter mit Verankerungselementen versehen ist,
- Fig. 2: ein distales Ende des Neurostimulationskatheters in weiter vereinfachter Darstellung und mit zwecks Verankerung abgespreizten Verankerungselementen,
- Fig. 3: das distale Ende des Neurostimulationskatheters nach einem galvanischen Auflösen der Verankerungselemente,
- Fig. 4, 5: jeweils ein distales Ende einer weiteren Ausführungsform eines erfindungsgemäßen Neurostimulationskatheters, bei welchem die Verankerungselemente jeweils lediglich abschnittsweise aus einem galvanischen Material gefertigt sind,
- Fig. 6, 7: jeweils ein distales Ende einer weiteren Ausführungsform eines erfindungsgemäßen Neurostimulationskatheters, bei welchem die Verankerungselemente mittels eines biokompatiblen Klebstoffs in einer angelegten Einführposition (Fig. 6) fixiert und mittels eines Vorspannelements in eine abgespreizte Verankerungsposition (Fig. 7) vorgespannt sind,
- Fig. 8, 9: jeweils ein distales Ende einer weiteren Ausführungsform eines erfindungsgemäßen Neurostimulationskatheters, wobei ein Verankerungselement als Schraubwendel gestaltet und vollständig aus einem galvanisch auflösbaren Material gefertigt ist,
- Fig. 10, 11: jeweils ein distales Ende einer weiteren Ausführungsform eines erfindungsgemäßen Neurostimulationskatheters, wobei das Verankerungselement wiederum als Schraubwendel gefertigt, aber lediglich abschnittsweise aus einem galvanischen Material gefertigt ist, und
- Fig. 12: in schematischer Blockdarstellung eine Ausführungsform eines offenbarungsgemäßen Verfahrens zum Verankern und Lösen eines Neurostimulationskatheters.

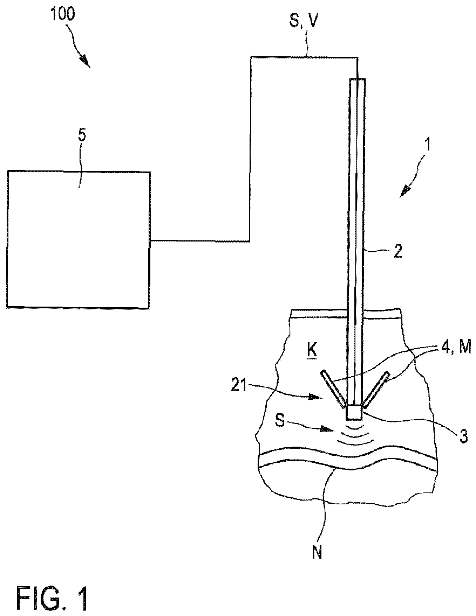

Gemäß Fig. 1 ist ein medizinisches System 100 zur Verwendung bei einer Schmerztherapie mittels elektrischer Neurostimulation vorgesehen. Das medizinische System 100 ist in Fig. 1 schematisch stark vereinfacht in einer exemplarischen Verwendungssituation dargestellt.

Das medizinische System 100 weist einen Neurostimulationskatheter 1 mit einem längserstreckten Katheterschaft 2 und wenigstens einer an dem Katheterschaft 2 angeordneten Stimulationselektrode 3 auf. Zudem weist das medizinische System 100 einen Signalgenerator 5 auf, der zum Erzeugen des elektrischen Stimulationssignals S eingerichtet ist.

In der exemplarisch gezeigten Verwendungssituation ist der Katheterschaft 2 in ein Körpergewebe K eines Patienten eingeführt. Dabei ist die Stimulationselektrode 3 in der Nähe eines Nervs N angeordnet. Das mittels des Signalgenerators 5 erzeugbare und mittels der Stimulationselektrode 3 abgebbare Stimulationssignal S ist dazu eingerichtet, eine Erregungsweiterleitung des Nervs N zu hemmen. Durch diese Hemmung wird das Schmerzempfinden des Patienten unterdrückt. Die Effektivität des Stimulationssignals S wird maßgeblich durch die Positionierung der Stimulationselektrode 3 in Relation zu dem Nerv N beeinflusst. Insbesondere eine proximale Dislokation des Katheterschafts 2 und damit der Stimulationselektrode 3 kann zu einer übermäßigen Vergrößerung des Abstands zwischen der Stimulationselektrode 3 und dem Nerv N und damit zu einer Beeinträchtigung der Schmerzunterdrückung führen. Im Übrigen ist eine solche Schmerztherapie per Neurostimulation oder auch Neuromodulation einer vorliegend zuständigen Fachperson prinzipiell bekannt. Auf weitere Einzelheiten hierzu muss daher an dieser Stelle nicht eingegangen werden.

Um eine ungewollte Dislokation der Stimulationselektrode 3 zu vermeiden, weist der Neurostimulationskatheter 1 wenigstens ein Verankerungselement 4 auf, das an dem Katheterschaft 2 befestigt ist.

Bei der gezeigten Ausführungsform sind zwei Verankerungselemente 4 vorhanden. Bei weiteren Ausführungsformen ist lediglich ein (einziges) Verankerungselement oder auch mehr als die vorliegenden zwei Verankerungselemente vorhanden. Der gebotenen Kürze wegen wird nachfolgend lediglich auf ein/das Verankerungselement 4 Bezug genommen. Die diesbezügliche Offenbarung gilt auch für das weitere (zweite) Verankerungselement und etwaige weitere Verankerungselemente.

Das Verankerungselement 4 ist zur Verankerung an dem Körpergewebe K eingerichtet. Dabei ist in Fig. 1 exemplarisch eine solche Verankerung gezeigt. Zwecks Verankerung wirkt das Verankerungselement 4 kraft- und/oder formschlüssig mit dem umgebenden Körpergewebe K zusammen. Hierdurch wird insbesondere einer proximalen Verlagerung des Katheterschafts 2 entgegengewirkt.

Nach Abschluss der eigentlichen Schmerztherapie muss der Neurostimulationskatheter 1 aus dem Körpergewebe K entfernt werden. Hierfür wird der Katheterschaft 2 zusammen mit der Stimulationselektrode 3 proximal aus dem Körpergewebe K herausgezogen. Zuvor muss die Verankerung mittels des Verankerungselements 4 gelöst werden. Zu diesem Zweck ist das Verankerungselement 4 wenigstens abschnittsweise aus einem galvanischen Material M gefertigt. Das galvanische Material M ist unter Einwirkung eines elektrischen Gleichspannungssignals V galvanisch auflösbar. Durch das Auflösen des galvanischen Materials M wird die Verankerung zwischen dem Verankerungselement 4 und dem Körpergewebe K gelöst.

Bei der gezeigten Ausführungsform ist der Signalgenerator 5 zum Erzeugen des Gleichspannungssignals S eingerichtet und mit dem Verankerungselement 4 verbunden. Die besagte Verbindung erfolgt über eine Signalleitung (ohne Bezugszeichen), die drahtgebunden oder drahtlos ausgeführt sein kann. Bei der gezeigten Ausführungsform wird auch das Stimulationssignal S über die besagte Signalleitung übertragen.

Im Übrigen sind die Stimulationselektrode 3 und das Verankerungselement 4 bei der gezeigten Ausführungsform an einem distalen Ende 21 des Katheterschafts 2 angeordnet. Der Katheterschaft ist zwischen dem distalen Ende 21 und einem proximalen Ende (ohne Bezugszeichen) längserstreckt, das in der exemplarisch gezeigten Verwendungssituation außerhalb des Körpergewebes K angeordnet ist.

Die in den Figuren gezeigte Positionierung des Verankerungselements 4 an dem distalen Ende 21 und in Relation zu der Stimulationselektrode 3 ist als rein exemplarisch und nicht maßstäblich zu verstehen. Um eine mechanische Reizung des Nervs N durch das Verankerungselement 4 zu vermeiden, kann es vorteilhaft sein, das Verankerungselement 4 in proximaler Richtung weiter versetzt zu der Stimulationselektrode 3 an dem Katheterschaft 2 anzuordnen, als dies in den Figuren schematisch gezeigt ist.

Der Katheterschaft 2 kann auch als Stimulatorschaft bezeichnet werden. Bei der in den Figuren gezeigten Ausführungsform ist die Stimulationselektrode 3 an dem Katheterschaft 2 ausgebildet. Mit anderen Worten: Der Katheterschaft selbst bildet die Stimulationselektrode. Umgekehrt kann auch davon gesprochen werden, dass die Stimulationselektrode längserstreckt ist und gleichsam den Katheterschaft/Stimulatorschaft bildet. Im einfachsten Fall sind Katheterschaft und Stimulationselektrode durch einen Draht gebildet.

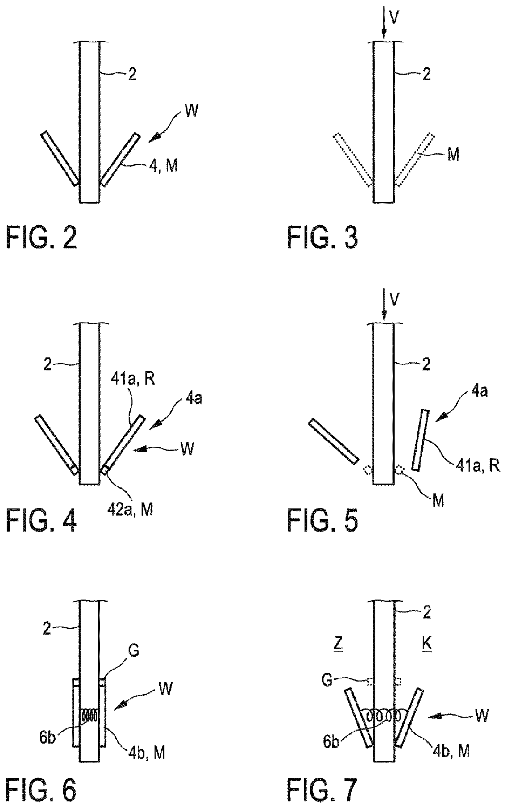

In den Fig. 2 und 3 ist das distale Ende 21 im Detail und unter Ausblendung der Stimulationselektrode 3 gezeigt. Dabei zeigt Fig. 2 eine Verankerungsposition mit (noch) nicht aufgelöstem Verankerungselement 4. In Fig. 3 ist das Verankerungselement 4 unter Einwirkung des Gleichspannungssignals V galvanisch aufgelöst. Dies wird in Fig. 3 durch die punktierte Umrisslinie symbolhaft verdeutlicht.

Bei der gezeigten Ausführungsform ist das galvanische Material M Zink. Bei in den Figuren nicht gezeigten Ausführungsformen handelt es sich bei dem galvanischen Material um Eisen, Nickel, Zinn und/oder Kupfer.

Bei der in den Fig. 1 bis 3 gezeigten Ausführungsform ist das gesamte Verankerungselement 4 aus dem galvanischen Material M gefertigt. Unter Einwirkung des Gleichspannungssignals V wird folglich das gesamte Verankerungselement 4 galvanisch aufgelöst.

Hierzu im Unterschied ist in den Fig. 4 und 5 eine Ausführungsform mit einem Verankerungselement 4a gezeigt, das lediglich abschnittsweise aus dem galvanischen Material M gefertigt ist. Im Speziellen weist das Verankerungselement 4a einen Verankerungsabschnitt 41a und einen Befestigungsabschnitt 42a auf. Der Befestigungsabschnitt 42a ist unmittelbar an dem Katheterschaft 2 befestigt. Der Verankerungsabschnitt 41a dient der eigentlichen Verankerung an dem Körpergewebe K und ist fest mit dem Befestigungsabschnitt 42a verbunden. Der Befestigungsabschnitt 42a ist deutlich kleiner als der Verankerungsabschnitt 41a. Der Befestigungsabschnitt 42a ist aus dem galvanischen Material M gefertigt. Der Verankerungsabschnitt 41a ist aus einem resorbierbaren Material R gefertigt. Bei dem resorbierbaren Material R kann es sich beispielsweise um einen resorbierbaren Kunststoff oder dergleichen handeln. Unter Einwirkung des Gleichspannungssignals V wird folglich lediglich der Befestigungsabschnitt 42a aufgelöst (siehe Fig. 5). Hierdurch wird der Verankerungsabschnitt 41a, d. h. der Rest des Verankerungselements 4a, von dem Katheterschaft 2 abgetrennt und verbleibt zunächst im Körpergewebe K. Durch die Fertigung aus dem erwähnten resorbierbaren Material R wird der Verankerungsabschnitt 41a mit der Zeit in dem Körpergewebe K aufgelöst/resorbiert.

Bei der Ausführungsform nach Fig. 1 bis 3 und bei der Ausführungsform der Fig. 4 und 5 ist das Verankerungselement 4 bzw. 4a jeweils ein Widerhakenelement W. Das Widerhakenelement W ist in der Verankerungsposition (Fig. 1, 2, 4) nach außen von dem Katheterschaft 2 abgespreizt. Mit anderen Worten: Das Widerhakenelement W steht zwecks Verankerung an dem Körpergewebe K von dem Katheterschaft 2 ab.

Das Verankerungselement, im Speziellen das Widerhakenelement, kann relativ zu dem Katheterschaft unbeweglich oder beweglich sein. Dabei ist in den Fig. 6 und 7 eine Ausführungsform mit relativbeweglichem Verankerungselement gezeigt.

Bei der erwähnten Ausführungsform nach den Fig. 6 und 7 ist das Verankerungselement 4b ebenfalls als Widerhakenelement W gestaltet. Das Widerhakenelement W ist zwischen einer Einführposition (Fig. 6) und einer Verankerungsposition (Fig. 7) relativ zu dem Katheterschaft 2 verlagerbar. In der Einführposition ist das Widerhakenelement W an den Katheterschaft 2 angelegt. In der Verankerungsposition ist das Widerhakenelement von dem Katheterschaft 2 abgespreizt. Dabei ist das Widerhakenelement W mittels eines Vorspannelements 6b ausgehend von der Einführposition in Richtung der Verankerungsposition elastisch vorgespannt. Zudem ist das Widerhakenelement W in der Einführposition fixiert. Zwecks Fixierung ist ein biokompatibler Klebstoff G vorhanden. Dessen in den Fig. 6 und 7 gezeigte Position ist als rein exemplarisch zu verstehen. Entsprechendes gilt für die Position und Gestaltung des Vorspannelements, das in den Fig. 6 und 7 beispielhaft als Wendelfeder gezeigt ist.

Der biokompatible Klebstoff G ist unter Einwirkung des Körpergewebes K, im Speziellen unter Einwirkung einer Zustandsgröße Z des Körpergewebes, auflösbar. Bei der Zustandsgröße Z handelt es sich um eine physikalische und/oder chemische Zustandsgröße, wie beispielsweise eine Feuchtigkeit, eine Temperatur und/oder einen pH-Wert des Körpergewebes K. Bei der gezeigten Ausführungsform ist der biokompatible Klebstoff G unter Einwirkung der Feuchtigkeit des Körpergewebes K auflösbar. Sobald der Katheterschaft 2 mitsamt dem per Klebstoff G in der Einführposition fixierten Verankerungselement 4b in das Körpergewebe K eingeführt ist, wird dessen Feuchtigkeit den Klebstoff G auflösen. Nach Auflösung des Klebstoffs G (siehe Fig. 7) bewirkt das Vorspannelement 6b eine selbsttätige Verlagerung des Verankerungselements 4b in Richtung der Verankerungsposition. Das Lösen der Verankerung erfolgt wie unter Bezugnahme auf die Fig. 1 bis 3 bzw. 4 und 5 beschrieben. Dabei kann das Verankerungselement 4b wiederum vollständig oder lediglich im Bereich eines Befestigungsabschnitts aus dem galvanischen Material M gefertigt sein.

Bei der gezeigten Ausführungsform ist der biokompatible Klebstoff G ein Zucker, im Speziellen aus Zuckermolekülen gebildet.

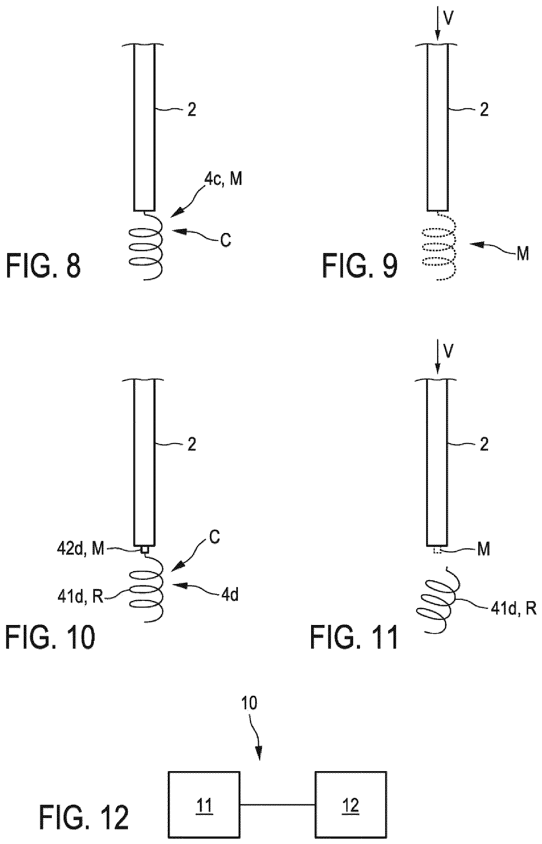

Bei den Ausführungsformen nach den Fig. 8 und 9 sowie den Fig. 10 und **11** sind die jeweiligen Verankerungselemente 4c, 4d als Schraubwendel C gestaltet. Die Schraubwendel C erstreckt sich ausgehend von dem distalen Ende des Katheterschafts 2 weiter in distale Richtung und ist zum Einschrauben in das Körpergewebe K eingerichtet. Es versteht sich, dass die in den Fig. 8 bis **11** gezeigte Formgebung der Schraubwendel C sowie deren relative Abmessungen im Vergleich zu dem Katheterschaft 2 als rein exemplarisch zu verstehen sind.

Bei der Ausführungsform nach den Fig. 8 und 9 ist das gesamte Verankerungselement 4c, d. h. die gesamte Schraubwendel C, aus dem galvanischen Material M gefertigt. Unter Einwirkung des Gleichspannungssignals V löst sich die gesamte Schraubwendel C auf (siehe Fig. 9).

Hierzu im Unterschied ist das Verankerungselement 4d bei der Ausführungsform nach den Fig. 10 und **11** lediglich abschnittsweise aus dem galvanisch auflösbaren Material M gefertigt. Dabei weist das Verankerungselement 4d in Form der Schraubwendel C wiederum einen Befestigungsabschnitt 42d und einen Verankerungsabschnitt 41d auf. Der Verankerungsabschnitt 41d bildet wenigstens den Großteil der Schraubwendel C. Der Befestigungsabschnitt 42d ist einends der Schraubwendel C angeordnet und unmittelbar an dem distalen Ende des Katheterschafts 2 befestigt. Der Verankerungsabschnitt 41d ist wiederum aus dem resorbierbaren Material R gefertigt. Unter Einwirkung des Gleichspannungssignals V wird lediglich der Befestigungsabschnitt 42d galvanisch aufgelöst, wodurch der Verankerungsabschnitt 41d von dem Katheterschaft 2 abgetrennt wird.

In Fig. 12 ist ein Verfahren zum Verankern und Lösen eines Neurostimulationskatheters gemäß den vorhergehenden Ausführungsformen schematisch stark vereinfacht dargestellt. Das Verfahren 10 sieht ein Verankern 11 des Katheterschafts vor, wobei der Katheterschaft mittels des Verankerungselements, das an dem Katheterschaft befestigt ist, an dem Körpergewebe verankert wird. Weiter sieht das Verfahren 10 ein Lösen 12 der Verankerung gemäß der vorhergehenden Beschreibung in Bezug auf die Ausführungsformen nach den Fig. 1 bis 11 vor. Folglich wird das elektrische Gleichspannungssignal V an das Verankerungselement angelegt, wodurch sich dieses wenigstens abschnittsweise (wie bei den Ausführungsformen nach den Fig. 4, 5 sowie 10 und 11) oder vollständig (wie bei den Ausführungsformen nach den Fig. 1 bis 3 sowie 8 und 9) auflöst und die Verankerung an dem Körpergewebe K gelöst wird.

## Patentansprüche

1. Neurostimulationskatheter (1) zur Verwendung bei einer Schmerztherapie, mit einem längserstreckten Katheterschaft (2) und wenigstens einer an dem Katheterschaft (2) angeordneten Stimulationselektrode (3), die zum Abgeben eines elektrischen Stimulationssignals (S) eingerichtet ist, wobei wenigstens ein Verankerungselement (4, 4a bis 4d) an dem Katheterschaft (2) befestigt ist, wobei das Verankerungselement (4, 4a bis 4d) zur Verankerung an einem den Katheterschaft (2) umgebenden Körpergewebe (K) eingerichtet ist,
**dadurch gekennzeichnet, dass** das Verankerungselement (4, 4a bis 4d) wenigstens abschnittsweise aus einem galvanischen Material (M) gefertigt ist, wodurch das Verankerungselement (4, 4a bis 4d) unter Einwirkung eines elektrischen Gleichspannungssignals (V) wenigstens abschnittsweise galvanisch auflösbar ist und damit die Verankerung an dem Körpergewebe (K) lösbar ist.

2. Neurostimulationskatheter (1) nach Anspruch 1, wobei das gesamte Verankerungselement (4, 4b, 4c) aus dem galvanischen Material (M) gefertigt ist.

3. Neurostimulationskatheter (1) nach Anspruch 1, wobei das Verankerungselement (4a, 4b, 4d) einen Verankerungsabschnitt (41a, 41d), der zum Zusammenwirken mit dem Körpergewebe (K) eingerichtet ist, und einen Befestigungsabschnitt (42a, 42d) aufweist, mittels dessen das Verankerungselement (4a, 4b, 4d) an dem Katheterschaft (2) befestigt ist, wobei der Befestigungsabschnitt (42a, 42d) aus dem galvanischen Material (M) gefertigt ist und der Verankerungsabschnitt (41, 41d) aus einem resorbierbaren Material (R) gefertigt ist.

4. Neurostimulationskatheter (1) nach einem der vorhergehenden Ansprüche, wobei das galvanische Material (M) Eisen, Nickel, Zinn, Kupfer und/oder Zink aufweist oder ist.

5. Neurostimulationskatheter (1) nach einem der vorhergehenden Ansprüche, wobei das Verankerungselement (4c, 4d) eine längserstreckte Schraubwendel (C) ist, die zum Einschrauben in das Körpergewebe (K) eingerichtet ist.

6. Neurostimulationskatheter (1) nach einem der Ansprüche 1 bis 4, wobei das Verankerungselement (4, 4a, 4b) ein Widerhakenelement (W) ist, das zum Verhaken an dem Körpergewebe (K) eingerichtet ist.

7. Neurostimulationskatheter (1) nach Anspruch 6, wobei das Widerhakenelement (W) relativ zu dem Katheterschaft (2) zwischen einer Einführposition und einer Verankerungsposition verlagerbar ist, und wobei das Widerhakenelement (W) in der Einführposition an dem Katheterschaft (2) angelegt und in der Verankerungsposition von dem Katheterschaft (2) abgespreizt ist.

8. Neurostimulationskatheter (1) nach Anspruch 7, wobei das Widerhakenelement (W) mittels eines Vorspannelements (6b) ausgehend von der Einführposition in Richtung der Verankerungsposition vorgespannt und mittels eines biokompatiblen Klebstoffs (G) in der Einführposition fixiert ist, wobei der biokompatible Klebstoff (G) unter Einwirkung einer physikalischen und/oder chemischen Zustandsgröße (Z) des Körpergewebes (K) löslich ist.

9. Neurostimulationskatheter (1) nach Anspruch 8, wobei die Zustandsgröße (Z) eine Feuchtigkeit, Fett, eine Temperatur und/oder ein pH-Wert ist.

10. Neurostimulationskatheter (1) nach Anspruch 8 oder 9, wobei der biokompatible Klebstoff (G) Zucker und/oder Stärke aufweist oder ist.

11. Medizinisches System (100) mit einem Neurostimulationskatheter (1) nach einem der vorhergehenden Ansprüche und mit einem Signalgenerator (5), der mit dem Verankerungselement (4, 4a bis 4d) verbunden und zum Erzeugen des elektrischen Gleichspannungssignals (V) eingerichtet ist.

12. Medizinisches System (100) nach Anspruch **11,** wobei der Signalgenerator (5) mit der wenigstens einen Stimulationselektrode (3) verbunden und zum Erzeugen eines gleichspannungsfreien Stimulationssignals (S) eingerichtet ist.

## Claims

1. Neurostimulation catheter (1) for use in pain therapy, having a longitudinally extended catheter shaft (2) and at least one stimulation electrode (3) that is arranged on the catheter shaft (2) and configured to deliver an electrical stimulation signal (S),
wherein at least one anchor element (4, 4a to 4d) is secured to the catheter shaft (2), the anchor element (4, 4a to 4d) configured to be anchored to body tissue (K) surrounding the catheter shaft (2),
**characterized in that** at least portions of the anchor element (4, 4a to 4d) being manufactured from a galvanic material (M), whereby at least said portions of the anchor element (4, 4a to 4d) are galvanically dissolvable under the action of a DC voltage signal (V), and hence the anchorage to the body tissue (K) can be released.

2. Neurostimulation catheter (1) according to Claim 1, wherein the entire anchor element (4, 4b, 4c) is manufactured from the galvanic material (M).

3. Neurostimulation catheter (1) according to Claim 1, wherein the anchor element (4a, 4b, 4d) comprises an anchor portion (41a, 41d), which is configured to interact with the body tissue (K), and a securing portion (42a, 42d), by means of which the anchor element (4a, 4b, 4d) is secured to the catheter shaft (2), with the securing portion (42a, 42d) being manufactured from the galvanic material (M) and the anchor portion (41, 41d) being manufactured from a resorbable material (R).

4. Neurostimulation catheter (1) according to any of the preceding claims, wherein the galvanic material (M) contains or is iron, nickel, tin, copper and/or zinc.

5. Neurostimulation catheter (1) according to any of the preceding claims, wherein the anchor element (4c, 4d) is a longitudinally extended helix (C), which is configured to be screwed into the body tissue (K).

6. Neurostimulation catheter (1) according to any of Claims 1 to 4, wherein the anchor element (4, 4a, 4b) is a barbed element (W), which is configured to be hooked into the body tissue (K).

7. Neurostimulation catheter (1) according to Claim 6, wherein the barbed element (W) is displaceable relative to the catheter shaft (2) between an insertion position and an anchor position, and wherein the barbed element (W) rests against the catheter shaft (2) in the insertion position and is splayed away from the catheter shaft (2) in the anchor position.

8. Neurostimulation catheter (1) according to Claim 7, wherein the barbed element (W) is prestressed by means of a prestressing element (6b) in the direction of the anchor position starting from the insertion position and is affixed in the insertion position by means of a biocompatible adhesive (G), the biocompatible adhesive (G) being soluble under the action of a physical and/or chemical property (Z) of the body tissue (K).

9. Neurostimulation catheter (1) according to Claim 8, wherein the property (Z) is a moisture, fat, a temperature and/or a pH value.

10. Neurostimulation catheter (1) according to Claim 8 or 9, wherein the biocompatible adhesive (G) contains or is sugar and/or starch.

11. Medical system (100) having a neurostimulation catheter (1) according to any of the preceding claims and having a signal generator (5) that is connected to the anchor element (4, 4a to 4d) and configured to create the DC voltage signal (V).

12. Medical system (100) according to Claim 11, wherein the signal generator (5) is connected to the at least one stimulation electrode (3) and configured to create a DC voltage-free stimulation signal (S).

## Revendications

1. Cathéter de neurostimulation (1) destiné à être utilisé dans une thérapie de la douleur, comprenant une tige de cathéter allongée (2) et au moins une électrode de stimulation (3) disposée sur la tige de cathéter (2), qui est conçue pour délivrer un signal de stimulation électrique (S), au moins un élément d'ancrage (4, 4a à 4d) étant fixé à la tige de cathéter (2), l'élément d'ancrage (4, 4a à 4d) étant conçu pour s'ancrer sur un tissu corporel (K) entourant la tige de cathéter (2),
**caractérisé en ce que** l'élément d'ancrage (4, 4a à 4d) est réalisé au moins partiellement à partir d'un matériau galvanique (M), de sorte que l'élément d'ancrage (4, 4a à 4d) est, sous l'action d'un signal de tension continue électrique (V), au moins partiellement soluble par voie galvanique, libérant ainsi l'ancrage sur le tissu corporel (K).

2. Cathéter de neurostimulation (1) selon la revendication 1, dans lequel l'ensemble de l'élément d'ancrage (4, 4b, 4c) est réalisé à partir du matériau galvanique (M).

3. Cathéter de neurostimulation (1) selon la revendication 1, l'élément d'ancrage (4a, 4b, 4d) comportant une partie d'ancrage (41a, 41d) qui est conçue pour coopérer avec le tissu corporel (K), et une partie de fixation (42a, 42d) au moyen de laquelle l'élément d'ancrage (4a, 4b, 4d) est fixé à la tige de cathéter (2), la partie de fixation (42a, 42d) étant réalisée à partir du matériau galvanique (M) et la partie d'ancrage (41, 41d) étant réalisée à partir d'un matériau résorbable (R).

4. Cathéter de neurostimulation (1) selon l'une quelconque des revendications précédentes, le matériau galvanique (M) comprenant ou étant du fer, du nickel, de l'étain, du cuivre et/ou du zinc.

5. Cathéter de neurostimulation (1) selon l'une quelconque des revendications précédentes, l'élément d'ancrage (4c, 4d) étant une hélice de vissage allongée (C) qui est conçue pour être vissée dans le tissu corporel (K).

6. Cathéter de neurostimulation (1) selon l'une quelconque des revendications 1 à 4, l'élément d'ancrage (4, 4a, 4b) étant un élément à ardillon (W) qui est conçu pour s'accrocher au tissu corporel (K).

7. Cathéter de neurostimulation (1) selon la revendication 6, l'élément à ardillon (W) étant déplaçable par rapport à la tige de cathéter (2) entre une position d'insertion et une position d'ancrage, et l'élément à ardillon (W), dans la position d'insertion, étant appliqué contre la tige de cathéter (2) et, dans la position d'ancrage, étant écarté de la tige de cathéter (2).

8. Cathéter de neurostimulation (1) selon la revendication 7, l'élément à ardillon (W) étant précontraint au moyen d'un élément de précontrainte (6b) à partir de la position d'insertion en direction de la position d'ancrage et étant fixé dans la position d'insertion au moyen d'un adhésif biocompatible (G), l'adhésif biocompatible (G) étant soluble sous l'action d'une grandeur d'état physique et/ou chimique (Z) du tissu corporel (K).

9. Cathéter de neurostimulation (1) selon la revendication 8, la grandeur d'état (Z) étant une humidité, une matière grasse, une température et/ou une valeur de pH.

10. Cathéter de neurostimulation (1) selon la revendication 8 ou 9, l'adhésif biocompatible (G) comprenant ou étant du sucre et/ou de l'amidon.

11. Système médical (100) comprenant un cathéter de neurostimulation (1) selon l'une quelconque des revendications précédentes et comprenant un générateur de signaux (5) qui est relié à l'élément d'ancrage (4, 4a à 4d) et est conçu pour générer le signal de tension électrique continue (V).

12. Système médical (100) selon la revendication 11, dans lequel le générateur de signaux (5) est relié à l'au moins une électrode de stimulation (3) et est conçu pour générer un signal de stimulation (S) dépourvu de tension continue.
